# EUROPÄISCHE PATENTSCHRIFT

(11) **EP 1 716 859 B1**
(45) Veröffentlichungstag und Bekanntmachung des Hinweises auf die Patenterteilung: **15.07.2009**
(21) Anmeldenummer: 06008325.0
(22) Anmeldetag: 21.04.2006
(51) Int. Cl.: A61K 36/185, A61K 36/45, A61K 36/48, A61K 36/63, A61P 1/00, A61P 1/02

(54) **Pflanzenextrakte gegen Halitosis, Gingivitis oder Parodontitis**
Plant extracts against halitosis, gingivitis or parodontitis
Extraits de plantes contre halitosis, gingivitis ou parodontitis

(30) Priorität: 26.04.2005 DE 102005019653
(43) Veröffentlichungstag der Anmeldung: 02.11.2006
(73) Patentinhaber: Henkel AG & Co. KGaA, 40589 Düsseldorf (DE)
(72) Erfinder: Bockmühl, Dirk, 42113 Wuppertal (DE); Boy, Julia, 42105 Wuppertal (DE); Barth, Adolf Peter, 42781 Haan (DE); Janßen, Frank, 40589 Düsseldorf (DE); Bernecker, Ullrich, 52074 Aachen (DE)

(56) Entgegenhaltungen:
- LOCOCK R A: "Bearberry" 2000, CANADIAN PHARMACEUTICAL JOURNAL 2000 CANADA, VOL. 133, NR. 5, PAGE(S) 38-40 , XP009068438 ISSN: 0828-6914 * das ganze Dokument *
- GLOECKL INGMAR ET AL: "Validated methods for direct determination of hydroquinone glucuronide and sulfate in human urine after oral intake of bearberry leaf extract by capillary zone electrophoresis" 25. September 2001 (2001-09-25), JOURNAL OF CHROMATOGRAPHY B, VOL. 761, NR. 2, PAGE(S) 261-266 , XP004318283 ISSN: 0378-4347 * das ganze Dokument *
- PAPER D H ET AL: "Bioavailability of drug preparations containing a leaf extract of Arctostaphylos uva-ursi (Uvae ursi folium)" 1993, PLANTA MEDICA, VOL. 59, NR. 7 SUPPL., PAGE(S) A589 , 41ST ANNUAL CONGRESS OF THE SOCIETY FOR MEDICINAL PLANT RESEARCH; DUESSELDORF, GERMANY; AUGUST 31-SEPTEMBER 4, 1993 , XP009068441 ISSN: 0032-0943 * das ganze Dokument *
- DATABASE WPI Section Ch, Week 200230 Derwent Publications Ltd., London, GB; Class B04, AN 2002-247100 XP002388543 & JP 2001 322943 A (KAO CORP) 20. November 2001 (2001-11-20)
- DATABASE WPI Section Ch, Week 200171 Derwent Publications Ltd., London, GB; Class B04, AN 2001-613511 XP002388544 & JP 2001 199864 A (KOSE KK) 24. Juli 2001 (2001-07-24)

## Beschreibung

Die vorliegende Erfindung betrifft Pflanzenextrakte zur Inhibierung von Enzymen, insbesondere zur Inhibierung der Methionin-Gamma-Lyase (METase), und zur Verhinderung oder Behandlung von Mundgeruch bzw. Halitosis, Gingivitis oder Parodontitis sowie pharmazeutische Zubereitungen, die diese Pflanzenextrakte enthalten.

Mundgeruch, auch Halitosis oder "Foetor ex ore" genannt, kann verschiedene Ursachen haben. Bei gesunden Menschen wird Mundgeruch jedoch in den meisten Fällen durch Bakterien im Mund- bzw. Rachenraum hervorgerufen, die Körperzellen oder Nahrungsreste verstoffwechseln, wobei als Abbauprodukte von Proteinen u.a. flüchtige Schwefelverbindungen ("volatile sulfur compounds", VSC) entstehen, die für den üblen Geruch verantwortlich gemacht werden.

Diese Schwefelverbindungen sind insbesondere: H₂S = Schwefelwasserstoff (zu etwa 30%), CH₃₋S-H= Methylmercaptan (zu etwa 60 %) und CH₃-S-CH₃ = Dimethylsulfid (zu etwa 10 %).

Die flüchtigen Schwefelverbindungen sind teilweise sehr aggressiv und können das Zahnfleisch und die Mundschleimhaut schädigen. So ist beispielsweise aus Ng W, Tonzetich J.; "Effect of hydrogen sulfide and methyl mercaptan on the permeability of oral mucosa"; J Dent Res. 1984 Jul;63(7):994-7; bekannt, daß Schwefelwasserstoff und Methylmercaptan die Durchlässigkeit der Mundschleimhaut erhöhen und so Zahnfleischerkrankungen Vorschub leisten können.

Zahnfleischerkrankungen stellen einen hohen Risikofaktor für die Gesundheit dar. Nach Untersuchungen der Weltgesundheitsorganisation (WHO) leiden über 50% der erwachsenen Bundesbürger an dringend behandlungsbedürftigen Parodontopathien.

Zahnfleischerkrankungen werden im Volksmund oft 'Parodontose' genannt. Dieser Ausdruck ist nicht korrekt, da die Endung "-ose" einen normalen, altersbedingten Rückgang eines Organs beschreibt. Richtig sind die Ausdrücke "Parodontitis" oder auch "entzündliche Parodontopathie". Sie ist neben der Karies die weit verbreiteste Erkrankung der Mundhöhle und tritt hauptsächlich bei Erwachsenen auf.

Unter Parodontitis versteht man eine durch Bakterien hervorgerufene entzündliche Veränderung des den Zahn umgebenden Gewebes, besonders des Kieferknochens. Eine Parodontitis (= mit Beteiligung der Zahnfleischtaschen und des Knochens) entwickelt sich immer aus einer Zahnfleischentzündung (Gingivitis) und befällt zuerst die der Reinigung am schlechtesten zugänglichen Zahnzwischenräume. Die sich in der Zahnfleischtasche befindlichen Bakterien wirken durch ihre Stoffwechselprodukte, insbesondere durch die von Ihnen produzierten VSC, destruktiv auf Zahnfleisch, Zahnsubstanz und Knochen ein und verstärken so ständig die bereits bestehende Parodontitis.

Dieses gesundheitliche Problem ist sehr weit verbreitet: Untersuchungen zeigen, daß etwa ab dem 35. Lebensjahr mehr Zähne durch Parodontopathien (Zahnfleischerkrankungen) als durch Karies verloren gehen.

Methionin-γ-Lyasen (METasen) gehören zu einer Enzymfamilie, die in Pro- und Eukaryonten (nicht jedoch in Säugern) vorkommen und die Umsetzung von Methionin zu Ammoniak, 2-Oxobutyrat und Methylmercaptan katalysieren. Als Cofaktor ist Pyridoxalphosphat notwendig, das an einer konservierten Region mit der Konsensussequenz [DQ]-[LIMVF]-X(3)-[STAGC]-[STAGCI]-T-K-[FYWQ]-[LIVMF]-X-G-[HQ]-[SGNH] bindet, wobei K als Pyridoxalphosphat-Bindestelle fungiert. X bezeichnet dabei beliebige Aminosäuren, während die in Klammern gesetzten Aminosäuren jeweils die für diese Position mögliche Alternativen beschreiben, d.h. [DQ] bedeutet, dass an dieser Position der Konsensussequenz entweder D oder Q stehen kann. Die METasen aus dem Protozoon Trichomonas vaginalis und den Bakterien Pseudomonas putida und Porphyromonas gingivalis wurden bereits beschrieben und charakterisiert. Für Fusobacterium nucleatum wurde durch die Annotation des Genoms das METase-Gen identifiziert.

Yoshimura M, Nakano Y, Yamashita Y, Oho T, Saito T und Koga T. beschreiben in "Formation of methyl mercaptan from L-methionine by Porphyromonas gingivalis"; Infect Immun. 2000 Dec;68(12):6912-6; eine L-Methionine-alpha-deamino-gamma-Mercaptomethan-Lyase (METase) aus P. gingivalis, die große Mengen Methylmercaptan produziert.

METase aus Bakterien der Mundhöhle wurde allerdings bislang nicht im Zusammenhang mit Halitosis beschrieben, wie der Publikation von Yoshimura M, Nakano Y und Koga T; "L-Methioninegamma-lyase, as a target to inhibit malodorous bacterial growth by trifluoromethionine"; Biochem Biophys Res Commun. 2002 Apr 12;292(4):964-8; Seite 964, rechte Spalte, erster Absatz; zu entnehmen ist.

Der Anmelderin ist es gelungen, die für das METase-Protein kodierende Sequenz des oralen Bakteriums Treponema denticola aufzuklären und damit erstmals zu zeigen, dass METasen auch in Spirochaeten vorkommen (WO 04/055202).

Die Basensequenz des mgl1-Gens aus T. denticola ist zu 64,5% identisch mit der Sequenz aus P. gingivalis und beinhaltet die Pyridoxal-Phosphat Binderegion, die allerdings im Unterschied zu den anderen Mgl-Proteinen an Pos. 209 ein Leu statt eines Val aufweist (CLUSTALW-Alignment):

Besonders interessant ist in diesem Zusammenhang der Austausch von Glu zu.Thr an Pos 320, das auf eine zusätzliche Phosphorylierungsstelle in dem Mgl-Protein aus *T.denticola* hinweisen könnte, die möglicherweise als Target für einen spezifischen Hemmstoff dienen kann.

Die Diagnose des Mundgeruchs erfolgt üblicherweise durch Bakterienkulturen, Sulfidnachweismethoden und organoleptische Methoden (Riechen), oder durch Meßgeräte, wie das im Internet unter http://www.hatithose.de/halimtr.htm (Stand 16.12.2002) beschriebene "Halimeter^{Ⓡ}".

Bislang wurde meistens versucht, Halitosis, Gingivitis oder Parodontitis durch bakterizide Wirkstoffe zu bekämpfen, die jedoch teilweise starke Nebenwirkungen haben. Ein anderer Ansatz sieht vor, die VSCs mithilfe von zinkhaltigen Verbindungen zu entfernen. Letzteres Verfahren hat allerdings allenfalls eine kurzfristige Abnahme der Geruchsbildung zur Folge und beugt Gingivitis oder Parodontitis nicht vor.

Es besteht daher ein erheblicher Bedarf an der Bereitstellung neuer Wirkstoffe gegen Halitosis, Gingivitis oder Parodontitis.

Überraschenderweise wurde nun in einem Screening-Verfahren unter Verwendung von Methionin-Gamma-Lyasen und Pflanzenextrakten gefunden, daß bestimmte Pflanzenextrakte Methionin-Gamma-Lyasen inhibieren können, so dass ein solches Screening-Verfahren verwendet werden kann, um Wirkstoffe gegen Halitosis, Gingivitis oder Parodontitis aufzufinden.

Das Screening-Verfahren zur Identifikation wirksamer Pflanzenextrakte gegen Halitosis, Gingivitis oder Parodontitis, ist dadurch gekennzeichnet, daß man
a) eine für eine Methionin-Gamma-Lyase codierende Nukleinsäure aus einem pro- oder eukaryotischen Organismus isoliert oder in vitro synthetisiert,
b) die für eine Methionin-Gamma-Lyase codierende Nukleinsäure kloniert und in geeigneten Wirtszellen exprimiert oder die Methionin-Gamma-Lyase in vitro synthetisiert,
c) die Methionin-Gamma-Lyase exprimierenden Wirtszellen in voneinander räumlich getrennten Ansätzen in Gegenwart einer Schwefelquelle und eines geeigneten Nachweisagens für Reaktionsprodukte durch Methionin-Gamma-Lyase katalysierter Reaktionen kultiviert; oder die Methionin-Gamma-Lyase in voneinander räumlich getrennten Ansätzen in Gegenwart von Methionin, Cystein oder Glutathion in ein in vitro Testsystem einbringt,
d) die Ansätze mit unterschiedlichen Pflanzenextrakten versetzt,
e) für jeden der voneinander räumlich getrennten Ansätze die Bildung von Reaktionsprodukten durch Methionin-Gamma-Lyase katalysierter Reaktionen bestimmt und so wirksame Pflanzenextrakte gegen Halitosis, Gingivitis oder Parodontitis identifiziert.

Vorteilhafterweise ermöglicht das Screening-Verfahren, wenn es mit Methionin-Gamma-Lyase produzierenden bakteriellen Wirtszellen durchgeführt wird, Pflanzenextrakte zu identifizieren, die die VSC-Bildung verhindern, ohne alle im Mundraum vorhandenen Mikroorganismen abzutöten. Man verwirft hierzu alle Pflanzenextrakte, die zwar die Bildung von VSC inhibieren, gleichzeitig aber die bakterielle Wirtszelle abtöten.

So können Nebenwirkungsfreie oder zumindest nebenwirkungsarme Wirkstoffe aufgefunden werden, die die natürliche und nützliche Keimbesiedelung der Mundhöhle nicht beschadigen.

Vorzugsweise isoliert oder synthetisiert man in Schritt a) eine für eine Methionin-Gamma-Lyase codierende Nukleinsäure, die ausgewählt ist unter METase-Genen aus dem Protozoon Trichomonas vaginalis und den Bakterien Treponema denticola, Pseudomonas putida, Clostridium sporogenes und Fusobacterium nucleatum, besonders bevorzugt Treponema denticola.

Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind beispielsweise aus dem "Lexikon der Biochemie", Spektrum Akademischer Verlag, Berlin, 1999, Band 1, S. 267-271 und Band 2; S. 227-229, bekannt.

Eine METase aus Clostridium sporogenes ist beispielsweise beschrieben in der Publikation von Kreis W und Hession C; "Isolation and purification of L-methionine-alpha-deamino-gammamercaptomethane-lyase (L-methioninase) from Clostridium sporogenes"; Cancer Res. 1973 Aug;33(8):1862-5.

Ebenfalls bevorzugt wird in Schritt a) eine für eine Methionin-Gamma-Lyase codierende Nukleinsäure isoliert oder synthetisiert, deren Nukleotidsequenz mit der in SEQ ID NO: 6 (METase-Gen aus Pseudomonas putida), SEQ ID NO: 5 (METase-Gen aus Fusobacterium nucleatum) oder, besonders bevorzugt, mit der in SEQ ID NO: 4 (METase-Gen aus Treponema denticola) von WO 04/055202 angegebenen Nukleotidsequenz zu mindestens 50 %, zu mindestens 75 %, zu mindestens 80 %, vorzugsweise mindestens 85 %, insbesondere mindestens 90 %, besonders bevorzugt mindestens 95 % und ganz besonders bevorzugt zu 100 % übereinstimmt.

Ebenfalls bevorzugt wird in Schritt a) eine Nukleinsäure isoliert oder synthetisiert, die für eine Methionin-Gamma-Lyase codiert, deren Aminosäuresequenz mit einer der in SEQ ID NO: 1 bis 3 von WO 04/055202 angegebenen Aminosäuresequenzen, besonders bevorzugt mit der in SEQ ID NO: 3 angegebenen, zu mindestens 70 %, vorzugsweise mindestens 80 %, insbesondere mindestens 90 %, besonders bevorzugt mindestens 95 % und ganz besonders bevorzugt zu 100 % identisch ist, oder deren Aminosäuresequenz einen Teil enthält, der mit einer der in SEQ ID NO: 1 bis 3 von WO 04/055202 angegebenen Aminosäuresequenzen zu mindestens 70 %, vorzugsweise mindestens 80 %, insbesondere mindestens 90 %, besonders bevorzugt mindestens 95 % und ganz besonders bevorzugt zu 100 % identisch ist.

Besonders bevorzugt wird in Schritt a) eine Nukleinsäure isoliert oder synthetisiert, die für eine Methionin-Gamma-Lyase codiert, deren Aminosäuresequenz einen Teil enthält, der mit der Konsensus-Sequenz [DQ]-[LIMVF]-X(3)-[STAGC]-[STAGCI]-T-K-[FYWQ]-[LIVMF]-X-G-[HQ]-[SGNH] zu mindestens 96 %, insbesondere zu mindestens 97 %, vorzugsweise zu mindestens 98 %, bevorzugt zu mindestens 99 %, besonders bevorzugt zu 100 % identisch ist.

Vorzugsweise wird in Schritt c) des Verfahrens eine Methionin-Gamma-Lyase exprimiert oder eingesetzt, deren Aminosäuresequenz mit der in SEQ ID NO: 3 (METase aus Pseudomonas putida), SEQ ID NO: 2 (METase aus Fusobacterium nucleatum), oder, besonders bevorzugt, mit der in SEQ ID NO: 1 (METase aus Treponema denticola) von WO 04/055202 angegebenen Aminosäuresequenz zu mindestens 50 %, vorzugsweise mindestens 70 %, insbesondere mindestens 80 %, besonders bevorzugt mindestens 90 % und ganz besonders bevorzugt zu 100 % übereinstimmt.

Geeignete Wirtszellen für die Expression in Schritt b) sind Mikroorganismen, insbesondere Bakterien, besonders bevorzugt E. coli.

Geeignete Nachweisagenzien für Reaktionsprodukte durch Methionin-Gamma-Lyase katalysierter Reaktionen sind beispielsweise Salze von Metallen, die mit Sulfidionen schwerlösliche Salze mit einem Löslichkeitsprodukt von <10 - 20 bilden, vorzugsweise ein Schwermetallsalz, das mit Sulfidionen als schwer lösliche Verbindung ausfällt. Geeignet ist zum Beispiel Bleiacetat (bevorzugte Konzentration: etwa 4 mg/l weiteste Konzentration: etwa 0,1 bis etwa 10 mg/l). Möglich sind ausserdem Salze von Hg, Ni, Sb, Sn, Zn, Ag, Cu, Co, Cd, wobei neben Pb Ag und Cu bevorzugt sind. Da die Löslichkeit von Sulfidionen im Sauren besonders gering ist, ist ein leicht saures Medium bevorzugt (pH kleiner oder gleich 7). Als Schwefelquelle können neben Methionin auch andere schwefelhaltige Aminosäuren (insb. Cystein) und organische Schwefelverbindungen (Glutathion) dienen (bevorzugte Konzentration: etwa 500 mg/l weiteste Konzentration: etwa 50 bis etwa 1000 mg/l). Von den Zellen aufgrund der METase-Akfivität aus Methionin als Schwefelquelle gebildete Sulfide werden als Bleisuffid ausgefällt und führen zu einer Schwarzfärbung der Kolonie.

Die Bestimmung von Reaktionsprodukten durch Methionin-Gamma-Lyase katalysierter Reaktionen kann durch eines der obengenannten Nachweisagenzien erfolgen oder z. B. durch Nachweis der bei folgender Reaktion gebildeten Produkte:

Methionin → Methylmercaptan + α-Ketobutyrat + Ammoniak (katalysiert METase).

Die Enzymaktivität der METase wird beispielsweise indirekt über die Umsetzung von α-Ketobutyrat und NADH₂ zu 2-Hydroxybutyrat und NAD⁺ im Photometer bei 340 nm (Absorptionsmaximum des NADH₂) über die Abnahme der Extinktion gemessen.

Um das METase-Protein zur Verwendung in technischen, kosmetischen oder pharmazeutischen Präparaten bereitzustellen, kommen alle Verfahren zur Herstellung rekombinanter Proteine in Betracht,

Erfindungsgemäß wurde gefunden, dass Pflanzenextrakte aus Bärentraube, Olive, Rotklee, Lindenblüten und Mate für den erfindungsgemäßen Zweck besonders gut geeignet sind, wobei Extrakte aus Bärentraube besonders bevorzugt sind.

Inhibitions-Untersuchungen mit weiteren Enzymen stützten hierbei die Vermutung, dass der Enzym-inhibitorische Effekt nicht auf die METasen allein beschränkt ist.

In WO021094300 werden bereits Zusammensetzungen zur Behandlung mukosaler Läsionen beschrieben, die Pflanzenextrakte aus Salix alba, Camellia sinensis oder Melissa officinalis enthalten können.

Ferner beschreibt R.A. Locock (Canadian Pharmaceutical Journal (2000), 133(5), 38-40) die Verwendung von Bärentraube zur Behandlung von Entzündungen des Urinaltraktes. Gloeckl et al. (Journal of Chromatography B (2001), 761(2), 261-266 sowie Paper et al. (Planta Medica (1993), 59(7), A589) berichten über den Einsatz von Bärentraube-Blattextrakten als Harntraktantiseptikum. Ferner wird in JP62148426 die Verwendung von Bärentraube-Extrakt zur Behandlung von Parodontose offenbart, wobei der Effekt zurückgeführt wird auf eine Inhibierung der Kollagenaktivität.

In JP2002104983 wird die Verwendung von Extrakten aus Tilia sp. zur Verhinderung der Anhaftung der Bakterien Streptococcus mutans und S. sobrinus und damit zur Verhinderung von Karies beschrieben. In BG107649 wird ein aufschäumendes pharmazeutisches Mittel zur oralen Applikation beschrieben, das unter anderem einen Extrakt aus Tilia grandifolia enthalten kann.

In WO04/062639 sind Kaugummizusammensetzungen beschrieben, die Extrakte aus Olea europensis oder Trifolium pratense enthalten können. WO05/063195 ist zu entnehmen, dass Zusammensetzungen, die u.a. einen Extrakt aus Olea europea enthalten können, zur Behandlung von Läsionen der Schleimhaut verwendet werden können.

JP2003073287 ist zu entnehmen, dass ein Extrakt aus Ilex latifolia in Zahnpasta, Gurgelwässer oder oralen Kühlungsmitteln eingesetzt werden kann.

Ein Gegenstand der vorliegenden Erfindung ist daher die Verwendung eines Pflanzenextraktes in einem kosmetischen Mittel zur Verhinderung oder Behandlung von Mundgeruch bzw. Halitosis, dadurch gekennzeichnet, dass der Pflanzenextrakt ausgewählt ist aus Extrakten von Arctostaphylos sp., Tilia sp., Olea sp., Trifolium sp. oder Ilex sp. oder Mischungen davon.

Bei dem Extrakt aus Arctostaphylos sp. handelt es sich vorzugsweise um einen Extrakt aus Arctostaphylos uva-ursi (Bärentraube). Mit Extrakten aus den Blättern der Bärentraube konnte mit 80 % Inhibition ein sehr guter Inhibitionswert erreicht werden. Verwendet wurden insbesondere Glycerin-Extrakte sowie Extrakte auf Maltodextrin/Silica-Tragern.

Bei dem Extrakt aus Tilia sp. handelt es sich vorzugsweise um einen Extrakt aus Tilia cordata. Mit einem Wasser/Propylenglykol-Extrakt aus der Lindenblüte konnte mit 34 % Inhibition der METase ein noch guter Wert erzielt werden.

Bei dem Extrakt aus Olea spec. handelt es sich vorzugsweise um einen Extrakt aus Olea europea (Olive). Es konnte hiermit eine 50 %ige Inhibition der METase erzielt werden. Verwendet wurde insbesondere ein Wasser/Ethanol-Extrakt aus Blättern auf Maltodextrin/Silica-Trägern.

Bei dem Extrakt aus Trifolium spec. (Klee) handelt es sich vorzugsweise um einen Extrakt aus Rotem Klee (Trifolium pratense). Es konnte hiermit eine 47 %ige Inhibition der METase erzielt werden. Verwendet wurde insbesondere ein Extrakt aus Blüten auf Silica-Tragern.

Bei dem Extrakt aus Ilex spec. handelt es sich vorzugsweise um einen Extrakt aus Ilex paraguariensis (Mate). Es konnte hiermit eine 57 %ige Inhibition der METase erzielt werden. Verwendet wurde insbesondere ein Propylenglykol/Wasser-Extrakt aus Blättern.

Die Herstellung der Extrakte kann grundsätzlich in jeder dem Fachmann bekannten Weise unter Verwendung jedes beliebigen Pflanzengewebes und unter Verwendung jedes beliebigen Extraktionsmittels erfolgen. So kann der Pflanzenextrakt beispielsweise durch Extraktion der gesamten Pflanze, durch Extraktion aus Blüten, Blättern, Frucht, Samen, Wurzeln und/oder durch Extraktion aus dem Meristem der Pflanze erfolgen.

Als Extraktionsmittel zur Herstellung der genannten Pflanzenextrakte können beispielsweise Wasser, Alkohole sowie deren Mischungen verwendet werden. Als Alkohole kommen beispielsweise niedere Alkohole wie Ethanol und Isopropanol, insbesondere aber auch mehrwertige Alkohole wie Ethylenglykol, Propylenglykol, Butylenglykol und Glycerin in Frage, und zwar sowohl als alleiniges Extraktionsmittel als auch in Mischung mit Wasser. So haben sich beispielsweise Pflanzenextrakte auf Basis von Wasser/Propylenglykol im Verhältnis 1:10 bis 10:1 als besonders geeignet erwiesen. Die Extraktion kann beispielsweise in Form von Wasserdampfdestillation durchgeführt werden. Gegebenenfalls kann auch eine Trockenextraktion und/oder eine Kohlendioxidextraktion erfolgen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch. Je nach Wahl der Extraktionsmittel kann es bevorzugt sein, den Pflanzenextrakt durch Zugabe eines Lösungsvermittlers zu stabilisieren. Als Lösungsvermittler geeignet sind z. B. Ethoxylierungsprodukte von gegebenenfalls gehärteten pflanzlichen und tierischen Olen. Bevorzugte Lösungsvermittler sind ethoxylierte Mono-, Di- und Triglyceride von C₈₋₂₂-Fettsäuren mit 4 bis 50 Ethylenoxid-Einheiten, z. B. hydriertes ethoxyliertes Castoröl, Olivenölethoxylat, Mandelölethoxylat, Nerzölethoxylat, Polyoxyethylenglykolcapryl-/- /caprinsäureglyceride, Polyoxyethylenglycerinmonolaurat und Polyoxyethylenglykolkokosfettsäure glyceride.

Die Pflanzenextrakte können, in einer erfindungsgemäß bevorzugten Ausführungsform, auch auf Träger aufgebracht werden, insbesondere um besser in Produkte eingearbeitet werden zu können. Erfindungsgemäß geeignete Träger sind beispielsweise Maltodextrin, Talk und Silica.

Die Pflanzenextrakte sind kommerziell beispielsweise von Cosmetochem (Deutschland) oder Cognis (Deutschland) erhältlich.

Ein weiterer Gegenstand der vorliegenden Erfindung sind pharmazeutische Zusammensetzungen, die einen Pflanzenextrakt ausgewählt aus Extrakten von Tilia sp., Trifolium sp. oder Ilex sp. oder Mischungen davon, gegebenenfalls in Träger-gebundener Form, enthalten, wobei es sich bei den pharmazeutischen Zusammensetzungen um solche zur Verwendung bei der Behandlung von Halitosis, Gingivitis oder Parodontitis handelt. Gegenstand der vorliegenden Erfindung ist ebenso die Verwendung der genannten Pflanzenextrakte zur Herstellung einer pharmazeutischen Zubereitung zur Behandlung oder Verhinderung von Halitosis, Gingivitis oder Parodontitis.

Weiterer Gegenstand der vorliegenden Erfindung ist eine pharmazeutische Zusammensetzung zur Verwendung bei der Behandlung von Halitosis, dadurch gekennzeichnet, dass sie einen Pflanzenextrakt ausgewählt aus Extrakten von Arctostaphylos sp. oder Olea sp. oder Mischungen davon enthalten

Bei der erfindungsgemäßen pharmazeutischen Zusammensetzung kann es sich um jede beliebige Darreichungsform handeln.

Bei der erfindungsgemäßen pharmazeutischen Zusammensetzung handelt es sich vorzugsweise um ein Mittel zur oralen Pflege, insbesondere um ein Mundwasser, ein Gel, eine flüssige Zahnputzlotion, eine steife Zahnpaste, einen Kaugummi, einen Gebissreiniger oder eine Prothesenhaftcreme. Ebenso erfolgt die erfindungsgemäße kosmetische Verwendung vorzugsweise in einer derartigen Ausführungsform.

### Mittel zur oralen Pflege

Die erfingdungsgemäß einzusetzenden Extrakte sind vorzugsweise in Mengen von 0,001-10 Gew.%, besonders bevorzugt von 0,01-2,0 Gew.-%, vor allem in Mengen von 0,05-1,0 Gew.-% in den Mund- oder Zahnpflegemitteln enthalten.

Hierzu ist es erforderlich, die erfindungsgemäßen Substanzen und/oder Extrakte in einen geeigneten Träger einzuarbeiten.

Als Träger können z.B. auch pulverförmige Zubereitungen oder wässrig-alkoholische Lösungen dienen, die als Mundwässer 0 bis 15 Gew.-% Ethanol, 1 bis 1,5 Gew.-% Aromaöle und 0,01 bis 0,5 Gew.-% Süßstoffe oder als Mundwasser-Konzentrate 15 bis 60 Gew.-% Ethanol, 0,05 bis 5 Gew.-% Aromaöle, 0,1 bis 3 Gew.-% Süßstoffe sowie ggf. weitere Hilfsstoffe enthalten können und vor Gebrauch mit Wasser verdünnt werden. Die Konzentration der Komponenten muss dabei so hoch gewählt werden, dass nach Verdünnung die genannten Konzentrationsuntergrenzen bei der Anwendung nicht unterschritten werden.

Als Träger können aber auch Gele sowie mehr oder weniger fließfähige Pasten dienen, die aus flexiblen Kunststoffbehältern oder Tuben ausgedrückt und mit Hilfe einer Zahnbürste auf die Zähne aufgetragen werden. Solche Produkte enthalten höhere Mengen an Feuchthaltemitteln und Bindemitteln oder Konsistenzreglern und Polierkomponenten. Darüber hinaus sind auch in diesen Zubereitungen Aromaöle, Süßstoffe und Wasser enthalten.

Als Feuchthaltemittel können dabei z.B. Glycerin, Sorbit, Xylit, Propylenglykole, Polyethenylenglycole oder Gemische dieser Polyole, insbesondere solche Polyethenylenglycole mit Molekulargewichten von 200 bis 800 (von 400 - 2000) verwendet werden enthalten sein. Bevorzugt ist als Feuchthaltemittel Sorbit in einer Menge von 25 - 40 Gew.-% enthalten.

Als Antizahnstein-Wirkstoffe und als Demineralisierungs-Inhibitoren können kondensierten Phosphate in Form ihrer Alkalisalze, bevorzugt in Form ihrer Natrium- oder Kaliumsalze enthalten sein. Die wäßrigen Lösungen dieser Phosphate reagieren aufgrund hydrolytischer Effekte alkalisch. Durch Säurezusatz wird der pH-Wert der erfindungsgemäßen Mund-, Zahn- und/oder Zahnprothesenpflegemittel auf die bevorzugten Werte von 7,5 - 9 eingestellt.

Es können auch Gemische verschiedener kondensierter Phosphate oder auch hydratisierte Salze der kondensierten Phosphate eingesetzt werden. Die spezifizierten Mengen von 2 - 12 Gew.-% beziehen sich jedoch auf die wasserfreien Salze. Bevorzugt ist als kondensiertes Phosphat ein Natrium- oder Kalium-tripolyphosphat in einer Menge von 5 - 10 Gew.-% der Zusammensetzung enthalten.

Ein bevorzugt enthaltener Wirkstoff ist eine karieshemmende Flüorverbindung, bevorzugt aus der Gruppe der Fluoride oder Monofluorophosphate in einer Menge von 0,1 - 0,5 Gew.-% Fluor. Geeignete Fluorverbindungen sind z.B. Natriummonofluorophosphat (Na₂PO₃F), Kaliummonofluorophosphat, Natrium- oder Kaliumfluorid, Zinnfluorid oder das Fluorid einer organischen Aminoverbindung.

Als Bindemittel und Konsistenzregler dienen z.B. natürliche und synthetische wasserlösliche Polymere wie Carragheen, Traganth, Guar, Stärke und deren nichtionogene Derivate wie z.B. Hydroxypropylguar, Hydroxyethylstärke, Celluloseether wie z.B. Hydroxyethylcellulose oder Methylhydroxypropylcellulose. Auch Agar-Agar, Xanthan-Gum, Pektine, wasserlösliche Carboxyvinylpolymere (z.B.- Carbopol^{®}-Typen), Polyvinylalkohol, Polyvinylpyrrolidon, höhermolekulare Polyethylenglykole (Molekulargewicht 10³ bis 10⁶ D). Weitere Stoffe, die sich zur Viskositätskontrolle eignen, sind Schichtsilikare wie z.B. Montmorillonit-Tone, kolloidale Verdickungskieselsäuren, z.B. Aerogel-Kieselsäure oder pyrogene Kieselsäuren.

Als Polierkomponenten können alle hierfür bekannten Poliermittel, bevorzugt aber Fällungs- und Gelkieselsäuren, Aluminiumhydroxid, Aluminiumsilicat, Aluminiumoxid, Aluminiumoxidtrihydrat, unlösliches Natriummetaphosphat, Calciumpyrophosphat, Calciumhydrogenphosphat, Dicalciumphosphat, Kreide, Hydroxylapatit, Hydrotalcite, Talkum, Magnesiumaluminiumsilicat (Veegum^{®}), Calciumsulfat, Magnesiumcarbonat, Magnesiumoxid, Natriumaluminiumsilikate, z.B. Zeolith A oder organische Polymere, z.B. Polymethacrylat, eingesetzt werden. Die Poliermittel werden vorzugsweise in kleineren Mengen von z.B. 1 - 10 Gew.-% verwendet.

Die erfindungsgemäßen Zahn- und/oder Mundpflegeprodukte können durch Zugabe von Aromaölen und Süßungsmitteln in ihren organoleptischen Eigenschaften verbessert werden. Als Aromaöle kommen alle für Mund-, Zahn- und/oder Zahnprothesenpflegemittel gebräuchlichen natürlichen und synthetischen Aromen in Frage. Natürliche Aromen können sowohl in Form der aus den Drogen isolierten etherischen Öle als auch der aus diesen isolierten Einzelkomponenten verwendet werden. Bevorzugt sollte wenigstens ein Aromaöl aus der Gruppe Pfefferminzöl, Krauseminzöl, Anisöl, Kümmelöl, Eukalyptusöl, Fenchelöl, Zimtöl, Geraniumöl, Salbeiöl, Thymianöl, Majoranöl, Basilikumöl, Citrusöl, Gaultheriaöl oder eine oder mehrere daraus isolierte synthetisch erzeugten Komponenten dieser Öle enthalten sein. Die wichtigsten Komponenten der genannten Öle sind z.B. Menthol, Carvon, Anethol, Cineol, Eugenol, Zimtaldehyd, Geraniol, Citronellol, Linalool, Salven, Thymol, Terpinen, Terpinol, Methylchavicol und Methylsalicylat. Weitere geeignete Aromen sind z.B. Menthylacetat, Vanillin, Jonone, Linalylacetat, Rhodinol und Piperiton. Als Süßungsmittel eignen sich entweder natürliche Zucker wie Sucrose, Maltose, Lactose und Fructose oder synthetische Süßstoffe wie z.B. Saccharin-Natriumsalz, Natriumcyclamat oder Aspartam.

Als Tenside sind dabei insbesondere Alkyl- und/oder Alkenyl-(oligo)-glycoside einsetzbar. Ihre Herstellung und Verwendung als oberflächenaktive Stoffe sind beispielsweise aus US-A-3 839 318, US-A-3 707 535, US-A-3 547 828 DE-A- 19 43 689, DE-A-20 36 472 und DE-A-30 01 064 sowie EP-A-77 167 bekannt. Bezüglich des Glycosidrestes gilt, daß sowohl Monoglycoside (x = 1), bei denen ein Pentose- oder Hexoserest glycosidisch an einen primären Alkohol mit 4 bis 16 C-Atomen gebunden ist, als auch oligomere Glycoside mit einem Oligomerisationsgrad x bis 10 geeignet sind. Der Oligomerisationsgrad ist dabei ein statistischer Mittelwert, dem eine für solche technischen Produkte übliche Homologenverteilung zugrunde liegt.

Bevorzugt eignet sich als Alkyl- und/oder Alkenyl-(oligo)-glycosid ein Alkyl- und/oder Alkenyl-(oligo)-glucosid der Formel RO(C₆H₁₀O)ₓ-H, in der R eine Alkyl- und/oder Alkenyl-gruppe mit 8 bis 14 C-Atomen ist und x einen Mittelwert von 1 bis 4 hat. Besonders bevorzugt sind Alkyloligoglucoside auf Basis von gehärtetem C_{12/14}-Kokosalkohol mit einem DP von 1 bis 3. Das Alkyl- und/oder Alkenyl-glycosid-Tensid kann sehr sparsam verwendet werden, wobei bereits Mengen von 0,005 bis 1 Gew.-% ausreichend sind.

Außer den genannten Alkylglucosid-Tensiden können auch andere nichtionische, ampholytische und kationische Tenside enthalten sein, als da beispielsweise sind: Fettalkoholpolyglycolethersulfate, Monoglyceridsulfate, Monoglyceridethersulfate, Mono- und/oder Dialkylsulfosuccinate, Fettsäureisethionate, Fettsäuresarcosinate, Fettsäuretauride, Fettsäureglutamate, Ethercarbonsäuren, Fettsäureglucamide, Alkylamido-betaine und/oder Proteinfettsäurekondensate, letztere vorzugsweise auf Basis von Weizenproteinen. Insbesondere zur Solubilisierung der meist wasserunlöslichen Aromaöle kann ein nichtionogener Lösungsvermittler aus der Gruppe der oberflächenaktiven Verbindungen erforderlich sein. Besonders geeignet für diesen Zweck sind z.B. oxethylierte Fettsäureglyceride, oxethylierte Fettsäuresorbitanpartialester oder Fettsäurepartialester von Glycerin- oder Sorbitan-Oxethylaten. Lösungsvermittler aus der Gruppe der oxethylierten Fettsäureglyceride umfassen vor allem Anlagerungsprodukte von 20 bis 60 Mol Ethylenoxid an Mono- und Diglyceride von linearen Fettsäuren mit 12 bis 18 C-Atomen oder an Triglyceride von Hydroxyfettsäuren wie Oxystearinsäure oder Ricinolsäure. Weitere geeignete Lösungsvermittler sind oxethylierte Fettsäuresorbitanpartialester; das sind bevorzugt Anlagerungsprodukte von 20 bis 60 Mol Ethylenoxid an Sorbitanmonoester und Sorbitandiester von Fettsäuren mit 12 bis 18 C-Atomen. Ebenfalls geeignete Lösungsvermittler sind Fettsäurepartialester von Glycerin- oder Sorbitan-Oxethylaten; das sind bevorzugt Mono- und Diester von C₁₂-C₁₈-Fettsäuren und Anlagerungsprodukten von 20 bis 60 Mol Ethylenoxid an 1 Mol Glycerin oder an 1 Mol Sorbit.

Die erfindungsgemäßen Mund-, Zahn- und/oder Zahnprothesenpflegemittel enthalten bevorzugt als Lösungsvermittler für gegebenenfalls enthaltene Aromaöle Anlagerungsprodukte von 20 bis 60 Mol Ethylenoxid an gehärtetes oder ungehärtetes Rizinusöl (d.h. an Oxystearinsäure- oder Ricinolsäure-triglycerid), an Glyzerin-mono- und/oder -distearat oder an Sorbitanmono- und/oder - distearat.

Weitere übliche Zusätze für die Mund-, Zahn- und/oder Zahnprothesenpflege mittel sind z.B.
- Pigmente, z.B. Titandioxid, und/oder Farbstoffe
- pH-Stellmittel und Puffersubstanzen wie z.B. Natriumbicarbonat, Natriumcitrat, Natriumbenzoat, Zitronensäure, Phosphorsäure oder saure Salze, z.B. NaH₂PO₄
- wundheilende und entzündungshemmende Stoffe wie z.B. Allantoin, Harnstoff, Panthenol, Azulen bzw. Kamillenextrakt
- weitere gegen Zahnstein wirksame Stoffe wie z.B. Organophosphonate, z.B. Hydroxyethandiphosphonate oder Azacycloheptandiphosphonat
- Konservierungsstoffe wie z.B. Sorbinsäure-Salze, p-Hydroxybenzoesäure-Ester.
- Plaque-Inhibitoren wie z.B. Hexachlorophen, Chlorhexidin, Hexetidin, Triclosan, Bromchlorophen, Phenylsalicylsäureester.
   In einer besonderen Ausführungsform ist die Zusammensetzung eine Mundspülung, ein Mundwasser, ein Prothesenreiniger oder ein Prothesenhaftmittel.

Für erfindungsgemäß bevorzugten Prothesenreiniger, insbesondere Prothesenreinigungstabletten und -pulver, eignen sich neben den schon genannten Inhaltsstoffen für die Mund-, Zahn- und/oder Zahnprothesenpflege zusätzlich noch Per-Verbindungen wie beispielsweise Peroxoborat, Peroxomonosulfat oder Percarbonat. Sie haben den Vorteil, dass sie neben der Bleichwirkung gleichzeitig auch desodorierend und/oder desinfizierend wirken. Der Einsatz solcher Per-Verbindungen in Prothesenreinigern beträgt zwischen 0,01 und 10 Gew.-%, insbesondere zwischen 0,5 und 5 Gew.-%.

Als weitere Inhaltsstoffe sind auch Enzyme, wie z.B. Proteasen und Carbohydrase, zum Abbau von Proteinen und Kohlenhydraten geeignet. Der pH-Wert kann zwischen pH 4 und pH 12, insbesondere zwischen pH 5 und pH 11 liegen.

Für die Prothesenreinigungstabletten sind zusätzlich noch weitere Hilfsstoffe notwendig, wie beispielsweise Mittel, die einen sprudelnden Effekt hervorrufen, wie z.B. CO₂ freisetzende Stoffe wie Natriumhydrogencarbonat, Füllstoffe, z.B. Natriumsulfat oder Dextrose, Gleitmittel, z.B. Magnesiumstearat, Fließregulierungsmittel, wie beispielsweise kolloidales Siliziumdioxid und Granuliermittel, wie die bereits erwähnten hochmolekularen Polyethylenglykole oder Polyvinylpyrrolidon.

Prothesenhaftmittel können als Pulver, Cremes, Folien oder Flüssigkeiten angeboten werden und unterstützen die Haftung der Prothesen.

Als Wirkstoffe sind natürliche und synthetische Quellstoffe geeignet. Als natürliche Quellstoffe sind neben Alginaten auch Pflanzengummen, wie z.B. Gummi arabicum, Traganth und Karaya-Gummi sowie natürlicher Kautschuk aufzufassen. Insbesondere haben sich Alginate und synthetische Quellstoffe, wie z.B. Natriumcarboxymethylcellulose, hochmolekulare Ethylenoxid-Copolymere, Salze der Poly(vinyl-ether-co-maleinsäure) und Polyacrylamide.

Als Hilfsstoffe für pastöse und flüssige Produkte eignen sich besonders hydrophobe Grundlagen, insbesondere Kohlenwasserstoffe, wie beispielsweise Weißes Vaselin (DAB) oder Paraffinöl.

Die folgenden Beispiele erläutern die Erfindung, ohne sie jedoch darauf einzuschränken:

### Beispiel 1: heterologe Expression des mgl1-Gens aus T. denticola

Die kodierende Sequenz des *mgl1*-Gens (METase-Gens) aus *T. denticola* wurde mithilfe von Datenbankvergleichen über PCR amplifiziert und in einen kommerziell verfügbaren Vektor (pGEM-TEasy, Promega) kloniert. Die Expression in *E. coli* kann wie folgt zum Screening nach erfindungsgemäßen wirksamen Pflanzenextrakten genutzt werden:

Die mit dem das *mgl1-*Gen enthaltenden Vektor transformierten E. coli-Zellen werden auf festen Nährmedien (z.B. LB-Medium, 1% Trypton, 0,5% Hefeextrakt, 0,5% NaCl, 1-2% Agar) angezogen. Zum Nachweis der Bildung von schwefelhaltigen Verbindungen wird dem Medium eine Schwefelquelle (Methionin; bevorzugte Konzentration: 350 mg/l weiteste Konzentration: 50-1000 mg/l) und ein Schwermetallsalz zugegeben, das mit Sulfidionen als schwer lösliche Verbindung ausfällt. Im Beispiel wurde dem Medium Bleiacetat zugegeben (bevorzugte Konzentration: 4 mg/l weiteste Konzentration: 0,1-10 mg/l).

Die Anzucht der Bakterien erfolgt bei 37°C für 2-7 Tage (bevorzugt 3 Tage) unter anaeroben Bedingungen in Petrischalen oder Multiwell-Platten. Auch aerobe Inkubation ist möglich, erfordert aber Inkubationsdauern von 5-10 Tagen.

Die Hemmwirkung des zu testenden Pflanzenextrakts wird durch die fehlende Schwarzfärbung der Kolonien sichtbar. Dabei ist von Vorteil, dass gleichzeitig eine wachstumshemmende Eigenschaft des Pflanzenextrakts überprüft werden kann, da die Expression des *mgl1*-Gens alleine keinen Einfluss auf das Wachstum hat.

### Beispiel 2: Enzymatische Tests mit aufgereinigten Mgl-Proteinen

Das *mg*/*1-Gen* aus *T. denticola* wird am 5'-Ende mit einer Sequenz versehen, die für sechs Histidin-Reste kodiert und in einen Expressionsvektor mit konstitutiv exprimiertem Promotor kloniert (pGEM). Die mit diesem Plasmid transformierten Zellen werden über Nacht in LB-Medium angezogen, anschließend wird eine weitere 50 ml Kultur mit einer OD₆₀₀=0,05 aus der vorherigen Kultur angeimpft und bei 37°C bis zu einer OD₆₀₀=0,2 angezogen. Die Kultur wird abzentrifugiert, in 1 ml Lysis-Puffer (50 mM NaH₂PO₄; 300 mM NaCl; 10 mM Imidazol; pH 8) resuspendiert und mindestens über Nacht bei -70°C eingefroren. Der Aufschluss erfolgt nach Lysozym-Zugabe (Endkonz. 1 mg/ml) und einem Inkubationsschritt von 30 min. auf Eis unter nativen Bedingungen mittels Ultraschall (insgesamt 2 min. mit je 5 sek. Pulsen und 5 sek. Pause). Das Lysat wird für 20-30 min. bei 13.000 Upm zentrifugiert und 600 µl des Überstandes über eine Ni-NTA-Säule (Qiagen) nach Herstellerangaben aufgereinigt.

Der eigentliche Enzymassay zum Screening nach erfindungsgemäßen wirksamen Substanzen wird wie folgt durchgeführt:

10-100 µg (1-1000µg) des aufgereinigten Enzyms werden in 50 mM Tris-HCl-Puffer (pH 7 (6-8)) zusammen mit 100 mM Methionin (10-1000 mM), 2 mM (1-10 mM) NADH, 100 µM Pyridoxalphosphat und 10U Lactatdehydrogenase ( Roche, bevorzugt aus Herzmuskelzellen, Isoenzym LDH-1) oder Hydroxybutyratdehydrogenase (TEso Diagnostics) bei 25°C inkubiert (in Eppendorf-Reaktionsgefäßen oder Mikrotiterplatten).

Die Enzymaktivität der METase kann indirekt über die Umsetzung von 2-Oxobutyrat und NADH₂ zu 2-Hydroxybutyrat und NAD⁺ im Photometer bei 340 nm (Absorptionsmaximum des NADH₂) über die Abnahme der Extinktion gemessen werden. Alternativ kann die Umsetzung in Küvetten auch unter direkter Beobachtung im Photometer gemessen werden. Inkubationsdauer 10-30 min bei 25°C.

Eine weitere Methode zur Bestimmung der Enzymaktivität ist die Reaktion des entstandenen Methylmercaptans mit 5,5'-Dithio-bis-2-Nitrobenzoesäure (DTNB; Sigma Aldrich). Dazu wird der Ansatz des oben beschriebenen Enzymassays in der Küvette mit DTNB (Endkonzentration 0,1 mM) versetzt und die Umsetzung zu einem Arylmercaptan bei 412 nm photometrisch verfolgt.

Mithilfe dieses Assays können Pflanzenextrakte auf ihre Hemmwirkung gegenüber der METase getestet werden, wichtig ist eine Kontrolle, bei der der Pflanzenextrakt auf seine Hemmwirkung auf LDH getestet wird. Dazu führt man obigen Versuch ohne Methionin und METase, jedoch in Anwesenheit von 2 mM Pyruvat durch. Etwaige Hemmwirkungen können durch Zugabe von Na₂SO₃ beseitigt werden.

### Beispiel 3: Nachweis der Aktivität von Pflanzenextrakten

Der Nachweis der Enzymaktivität von Mgl1 kann über eine Farbreaktion geführt werden, in der das aus Methionin freigesetzte Methylmercaptan durch 5,5'-dithiobis(2-nitrobenzoesäure) (DTNB) nachgewiesen wird. DTNB wird hierbei durch Methylmercaptan zu der gelben Substanz Mercaptonitrobenzoesäure MNB umgesetzt, deren Entstehung bei 412 nm im Photometer verfolgt werden kann(Abb.1)
Entsprechend wurden Hemmstoffe von Mgl1 mithilfe dieses Assays getestet, indem 10 µl Proteinextrakt aus Mgl1-exprimierenden Stämmen von E. coli mit 5 µl 2 mM DTNB, 10 µl 100 mM Methionin, 10 µl 1 M Tris pH8, 45 µl Wasser, 10 µl 100 µM Pyridoxalphosphat und Wirkstoff (wässrige Extrakte 1%; Trockenextrakte 0,1%) versetzt wurden. Nach 120 min wurde die Extinktion bei 412 nm gemessen und mit einer Kontrolle ohne Wirkstoff verglichen.

**Tabelle 1: Inhibitionswerte**

| Extrakt | Konzentration [%] E412nm | | Kontrolle | Restaktivität [%] |
|---|---|---|---|---|
| Bärentraube | 1 | 0,1488 | 0,7450 | 20 |
| Weidenrinde <*> | 1 | 0,1425 | 0,7450 | 19 |
| Weintraube <*> | 0,1 | 0,3661 | 0,705 | 52 |
| Olive | 0,1 | 0,3537 | 0,705 | 50 |
| Rotklee | 0,1 | 0,3708 | 0,705 | 53 |
| Grüner Tee <*> | 1 | 0,4626 | 0,7814 | 59 |
| Weißer Tee <*> | 1 | 0,379 | 0,7851 | 48 |
| Lindenblüten | 1 | 0.5879 | 0,8899 | 66 |
| Mate | 1 | 0,3857 | 0.8899 | 43 |
| Melisse <*> | 1 | 0,3947 | 0,8899 | 44 |

| | | | | |
|---|---|---|---|---|
| <*> nicht erfindungsgemäß | | | | |

### Sequenzen:

Hinsichtlich der Sequenzen der vorzugsweise verwendbaren METasen und der für diese Enzyme kodierenden Nukleinsäuren wird auf die Offenlegungsschrift WO 04/055202 verwiesen.

## Patentansprüche

1. Verwendung eines Pflanzenextraktes in einem kosmetischen Mittel zur Verhinderung oder Behandlung von Halitosis, **dadurch gekennzeichnet, dass** der Pflanzenextrakt ausgewählt ist aus Extrakten von Arctostaphylos sp., Tilia sp., Olea sp., Trifolium sp. oder Ilex sp. oder Mischungen davon.

2. Verwendung nach Anspruch 1, **dadurch gekennzeichnet, dass** es sich um ein Mittel zur oralen Pflege handelt.

3. Pharmazeutische Zusammensetzung zur Verwendung bei der Behandlung von Halitosis, Gingivitis oder Parodontitis enthaltend einen Pflanzenextrakt ausgewählt aus Extrakten von Tilia sp., Trifolium sp. oder Ilex sp. oder Mischungen davon.

4. Pharmazeutische Zusammensetzung zur Verwendung bei der Behandlung von Halitosis enthaltend einen Pflanzenextrakt ausgewählt aus Extrakten von Arctostaphylos sp. oder Olea sp. oder Mischungen davon.

5. Pharmazeutische Zusammensetzung nach Anspruch 3 oder 4, **dadurch gekennzeichnet, dass** es sich um ein Mundwasser, ein Gel, eine flüssige Zahnputzlotion, eine steife Zahnpaste, einen Kaugummi, einen Gebissreiniger oder eine Prothesehaftcreme handelt.

6. Verwendung eines Pflanzenextraktes ausgewählt aus Extrakten von Tilia sp., Trifolium sp. oder Ilex sp. oder Mischungen davon zur Herstellung einer pharmazeutischen Zusammensetzung zur Behandlung oder Verhinderung von Halitosis, Gingivitis oder Parodontitis.

## Claims

1. The use of a plant extract in a cosmetic agent for preventing or treating halitosis, **characterized in that** the plant extract is selected from extracts of *Arctostaphylos sp., Tilia sp., Olea sp., Trifolium sp.* or *Ilex sp.* or mixtures thereof.

2. The use according to claim 1, **characterized in that** it is an agent for oral care.

3. A pharmaceutical composition intended for use in the treatment of halitosis, gingivitis or parodontosis, containing an plant extract selected from extracts of *Tilia sp., Trifolium sp.* or *Ilex sp.* or mixtures thereof.

4. A pharmaceutical composition intended for use in the treatment of halitosis, containing an plant extract selected from extracts of *Arctostaphylos sp. or Olea sp.,* or mixtures thereof.

5. The pharmaceutical composition according to claim 3 or 4, **characterized in that** it is a mouthwash, a gel, a liquid dental cleaning lotion, a stiff tooth paste, a chewing gum, a denture cleaner or a prosthesis adherence cream.

6. The use of a plant extract selected from extracts of *Tilia sp., Trifolium sp.* or *Ilex sp.* or mixtures thereof, for the preparation of a pharmaceutical composition for treating or preventing halitosis, gingivitis or parodontosis.

## Revendications

1. Utilisation d'un extrait végétal dans un agent cosmétique pour empêcher ou traiter l'halitose, **caractérisée en ce que** l'extrait végétal est choisi parmi les extraits d'Arctostaphylos sp., Tilia sp., Olea sp., de Trifolium sp. ou d'Ilex sp. ou leurs mélanges.

2. Utilisation selon la revendication 1, **caractérisée en ce qu'**il s'agit d'un agent pour les soins buccaux.

3. Préparation pharmaceutique destinée à une utilisation lors du traitement de l'halitose, de la gingivite ou de la parodontite, contenant un extrait végétal choisi parmi les extraits de Tilia sp., de Trifolium sp. ou d'Ilex sp. ou leurs mélanges.

4. Préparation pharmaceutique destinée à une utilisation lors du traitement de l'halitose contenant un extrait végétal choisi parmi les extraits d'Arctostaphylos sp. ou d'Olea sp. ou leurs mélanges.

5. Préparation pharmaceutique selon la revendication 3 ou 4, **caractérisée en ce qu'**il s'agit d'un bain de bouche, d'un gel, d'une lotion dentifrice liquide, d'une pâte dentaire épaisse, d'un chewing-gum, d'un agent de nettoyage pour dentiers ou d'une crème adhésive de prothèse.

6. Utilisation d'un extrait végétal choisi parmi les extraits de Tilia sp., de Trifolium sp. ou d'Ilex sp. ou leurs mélanges pour la préparation d'une préparation pharmaceutique destinée au traitement ou à la prévention de l'halitose, de la gingivite ou de la parodontite.
